# EUROPEAN PATENT APPLICATION

(11) **EP 3 235 461 A1**
(43) Date of publication of application: **25.10.2017**
(21) Application number: 15869629.4
(22) Date of filing: 02.10.2015
(51) Int. Cl.: A61B 90/00, A61B 1/00, B25J 15/04

(54) **MANIPULATOR SYSTEM**

(30) Priority: 15.12.2014 JP 2014252686
(71) Applicant: Olympus Corporation, Hachioji-shi, Tokyo 192-8507 (JP)
(72) Inventor: KOMURO, Takahiro, Hachioji-shi, Tokyo 192-8507 (JP)
(74) Representative: Gunzelmann, Rainer
(86) International application number: PCT/JP2015/078103
(87) International publication number: WO 2016/098421

(57) **Abstract**

The invention has for its object to provide a manipulator system that has no or little influences on the surroundings even when a treatment tool is engaged with a drive unit after the insertion of the treatment tool through the body cavity, thereby allowing for unerring engagement of the treatment tool with the drive unit.

The manipulator system 1 includes a treatment tool 3, a first engagement portion 41a attached to the treatment tool 3 and formed on each of first driving force transmission members 41 to which associated driving forces are transmitted from a plurality of driving members 21, and a second engagement portion 42a formed on each of a plurality of second driving force transmission members 42 for transmission of associated driving forces to the plurality of first driving force transmission members 41. Mounting and demounting of the first on and out of the second engagement portions are separately sensed by an engagement sensor unit, and when the engagement of the first 41a with the second engagement portion 42a is sensed, there is deactivation of the second driving force transmission member 42 of the plurality of second driving force transmission members 42, which member has the sensed engagement portion 42a.

## Description

### Technical Field

The present invention relates to a manipulator system that is inserted through the body cavity of a patient for surgical operation or the like to view, and apply treatments or the like to, the patient's body cavity.

### Background Art

So far there has been medical equipment widely used in which a treatment tool is inserted through the body cavity of a patient and the distal end of the treatment tool is towed as by a wire to view, and apply treatments to, organs in the body cavity. Such a treatment tool must be replaced for each patient or treatment; so it is configured to be attached to or detached from a drive unit. In particular, it is also configured to be rotatable such that upon engagement of the treatment tool with the driver unit, an engagement portion on the drive unit side rotates relative to an engagement portion on the treatment tool side for automatic fitting and further rotates to confirm that engagement later.

Patent Literature 1 discloses a technology for engagement of the treatment tool with the drive unit at the time when the distal joint assembly of the treatment tool is outside the body of the patient.

### Citation List

### Patent Literature

Patent Literature 1: WO2010/126128

### Summary of Invention

### Technical Problem

With a prior art structure such as that of an endoluminal therapeutic device wherein after insertion of the treatment tool into the body of the patient, the treatment tool is engaged with the drive unit, however, the treatment tool may possibly be put into operation in the body cavity as the drive unit is driven after that engagement, resulting in interferences with body tissues.

With such a prior art problem in mind, an object of the invention is to provide a manipulator system that has no or little influences on the surroundings to allow for unerring engagement of the treatment tool with the drive unit even when the treatment tool is engaged with the drive unit after insertion of the treatment tool in the body cavity.

### Solution to Problem

According to one embodiment of the invention, a manipulator system includes,
a drive unit including a plurality of driving members for generating driving force,
a treatment tool that is driven by the drive unit,
an attachment/detachment mechanism for attaching and detaching the drive unit on and out of the treatment tool, and
a control unit for gaining control of the drive unit, characterized in that:
the attachment/detachment mechanism includes:
   a plurality of first driving force transmission members which are attached on the treatment tool and to which associated driving forces are transmitted from the plurality of driving members,
   a plurality of second driving force transmission members which are attached on the driving members and transmit associated driving forces to the plurality of first driving force transmission members, and
   an engagement sensor unit that is capable of separately sensing attaching and detaching of a first engagement portion formed on each of the plurality of first driving force transmission members on and out of a second engagement portion formed on each of the plurality of second driving force transmission members, and
   the control unit is configured such that when the engagement sensor unit senses engagement of the first engagement portion formed on each of the plurality of first driving force transmission members with the second engagement portion formed on each of the plurality of second driving force transmission members, there is deactivation of the driving member for driving the second driving force transmission member of the plurality of second driving force transmission members, which member has the sensed second engagement portion.

A manipulator system according to one embodiment of the invention includes:
a unit body for holding the drive unit,
a case for holding the treatment tool, and
a mount sensor unit for sensing mounting of the case on the unit body, wherein:
   the control unit is configured such that when the mount sensor unit senses mounting of the case on the unit body, the drive unit is activated.

In a manipulator system according to one embodiment of the invention, the attachment/detachment mechanism includes an intermediate member that includes a first intermediate engagement portion for engagement with the first engagement portion and a second intermediate engagement portion for engagement with the second engagement portion and is attachably/detachably located between the first driving force transmission members and the second driving force transmission members.

In a manipulator system according to one embodiment of the invention, the engagement sensor unit includes:
a first electrically conductive portion located in the first engagement portion,
a second electrically conductive portion located in the second engagement portion, and
a current sensor for sensing currents through the first and second electrically conductive portions.

In a manipulator system according to one embodiment of the invention, there is a fuse inserted through the first electrically conductive portion

In a manipulator system according to one embodiment of the invention, the intermediate member further includes a third electrically conductive portion interposed between the first and second intermediate engagement portions, and when the first engagement portion engages with the first intermediate engagement portion and the second engagement portion engages with the second intermediate engagement portion, it causes the third electrically conductive portion to make a connection between the first and second electrically conductive portions.

In a manipulator system according to one embodiment of the invention, the intermediate member further includes a resistance inserted between two third electrically conductive portions interposed between at least two first intermediate engagement portions and at least two second intermediate engagement portions, and
a voltage is applied on one of the second electrically conductive portions on the current sensor unit side and the other is connected to a positive terminal and a negative terminal of an A/D convertor with a sensing resistance Ra inserted between the positive terminal and the negative terminal.

In a manipulator system according to one embodiment of the invention, the engagement sensor unit includes:
a moving member located within the second engagement portion,
an elastic member for supporting the moving member relative to the second driving force transmission members,
a first electrically conductive portion located in the moving member,
a second electrically conductive portion located in the second engagement portion, and
a current sensor for sensing currents passing through the first and second electrically conductive portions.

A medical system according to one embodiment of the invention is characterized by including:
a master input including a master arm for issuing an operation command, and
a slave manipulator including a slave arm and the aforesaid manipulator system, wherein the slave arm is remotely controlled in such a way as to keep track of operation of the master arm.

### Advantageous Effects of Invention

According to the manipulator system described herein, it is possible to achieve unerring engagement of the treatment tool with the drive unit.

### Brief Description of Drawings

Fig. 1 is illustrative of the manipulator system according to one embodiment.
Fig. 2 is illustrative of the first engagement portion in the manipulator system according to one embodiment.
Fig. 3 is illustrative of the second engagement portion in the manipulator system according to one embodiment.
Fig. 4 is a control flowchart for the manipulator system according to one embodiment.
Fig. 5 is illustrative of the manipulator system 1 of the first example according to one embodiment in a disengaged state.
Fig. 6 is illustrative of the manipulator system 1 of the first example according to one embodiment in an engaged state.
Fig. 7 is illustrative of the manipulator system of the second example according to one embodiment in a disengaged state.
Fig. 8 is illustrative of the manipulator system of the second example according to one embodiment in an engaged state.
Fig. 9 is illustrative of the manipulator system of the third example according to one embodiment in an engaged state.
Fig. 10 is illustrative of one example of the intermediate member in the manipulator system according to the third example according to one embodiment.
Fig. 11 is illustrative of another example of the intermediate member in the manipulator system according to the third example according to one embodiment.
Fig. 12 is illustrative of the manipulator system of the fourth example according to one embodiment in an engaged state.
Fig. 13 is illustrative of the manipulator system of the fifth example according to one embodiment in an engaged state.
Fig. 14 is illustrative of the manipulator system of the sixth example according to one embodiment in an engaged state.
Fig. 15 is illustrative of the medical system according to one embodiment.
Fig. 16 is illustrative of the arrangement or structure of the insert assembly according to one embodiment.

### Description of Embodiments

Some embodiments will now be explained.

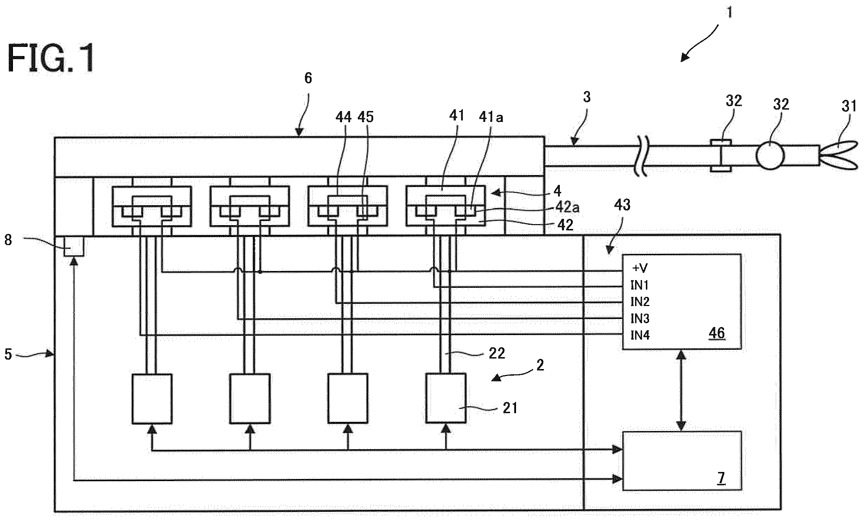

Fig. 1 is illustrative of the manipulator system according to one embodiment.

A manipulator system 1 according to one embodiment includes a drive unit 2 that includes a plurality of driving members 21, each generating driving force, a treatment tool 3 that is driven by the drive unit 2, an attachment/detachment mechanism 4 for attaching and detaching the drive unit 2 to and from the treatment tool 3, and a control unit 7 for gaining control of the drive unit 2.

The drive unit 2 includes the driving members 21 held at a unit body 5 and output shafts 22, each being configured to produce the driving force of each driving member 21. While there are four driving members 21 and four output shafts 22 provided in the drive unit 2 according to one embodiment, it is to be noted that at least two driving members and at least two output shafts may be provided. The respective driving members 21 are separately controlled by the control unit 7.

The treatment tool 3 is held in a case 6. The treatment tool 3 includes a grip portion 31 operating at its distal end and a plurality of bendable joints 32. The grip portion 31 and the plurality of joints 32 are each driven by different driving members 21 via a wire or the like.

The attachment/detachment mechanism 4 includes a plurality of first driving force transmission members 41 which are attached to the treatment tool 3 and to which associated driving forces are transmitted from the plurality of driving members 21, a plurality of second driving force transmission members 42 which are attached to the output shafts 22 of the driving members 21 for transmitting associated driving forces to the plurality of first driving force transmission members 42, and an engagement sensor unit 43 capable of separately sensing attaching and detaching of first engagement portions 41a, each one formed on each of the plurality of first driving force transmission members 41, on and from second engagement portions 42a, each one formed in each of the plurality of second driving force transmission members 42.

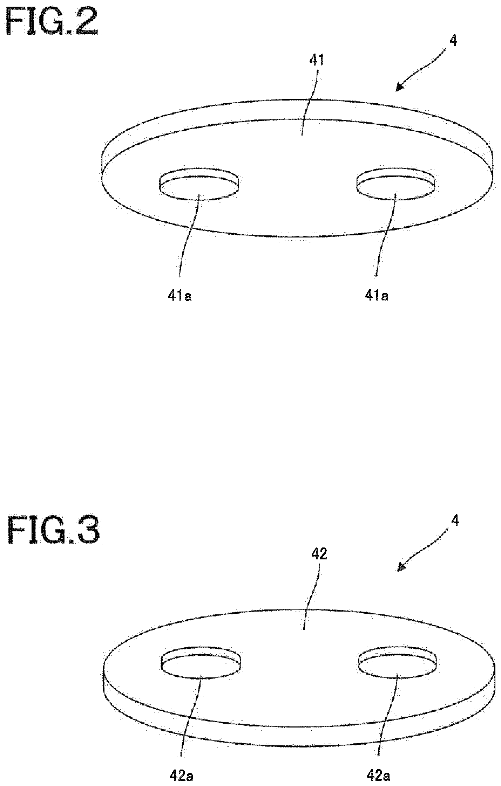

Fig. 2 shows the first engagement portion in the manipulator system according to one embodiment, and Fig. 3 shows the second engagement portion in the manipulator system according to one embodiment.

In one embodiment, the first and second engagement portions 41a and 42a are configured to be convex and concave, respectively, so that the first engagement portion 41a is fitted in the second engagement portion 42a. Note here that the first and second engagement portions 41a and 42a may be configured to be the opposite: concave and convex, respectively, and that they may have another structure provided that they can co-rotate during engagement.

The engagement sensor unit 43 includes a first electrically conductive portion 44 located in the first driving force transmission member 41, a second electrically conductive portion 45 located in the second driving force transmission member 42, and a current sensor 46 capable of sensing currents passing through the first 44 and the second electrically conductive portion 45. Upon engagement of the first 41a with the second electrically conductive portion 42a, there is a connection made between the first 44 and the second electrically conductive portion 45. The current sensor 46 is configured to sense currents passing as a result of connection of the first 44 to the second electrically conductive portion 45. The current sensor 46 is capable of separately sensing that the plurality of first electrically conductive portions 44 are each connected to the plurality of second electrically conductive portions 45. While the engagement sensor unit 43 according to this embodiment is configured to sense the engagement of the first 41a with the second electrically conductive portion 42a by currents passing through them, it is to be understood that a contact or distortion sensor may also be used as an alternative.

The control unit 7 gains control over the plurality of driving members 21. At the time when the engagement sensor unit 43 senses the engagement of the first 41a with the second engagement portion 42a, the control unit 7 operates to deactivate the driving of the driving members 21 of the plurality of second driving force members 42 which has the sensed second engagement portion 42a. And once the engagement of all the first and second engagement portions 41a and 42a has been sensed, the driving of all the driving members 21 is deactivated. In the manipulator system 1 according to this embodiment, this state is defined as an initial state from which treatments are preferably started.

It is here to be noted that the driving of the first driving member 21 for engagement of the first 41a with the second engagement portion 42a may be activated as by a switch button or, alternatively, the drive unit 2 may be driven after the mounting of the case 6 on the unit body 5 has been sensed by a mount sensor unit 8.

The initial control flow run by the control unit 7 in the manipulator system 1 according to the embodiment described here is now explained with reference to control of one driving member 21; however, control is actually gained of all of the plurality of driving members 21.

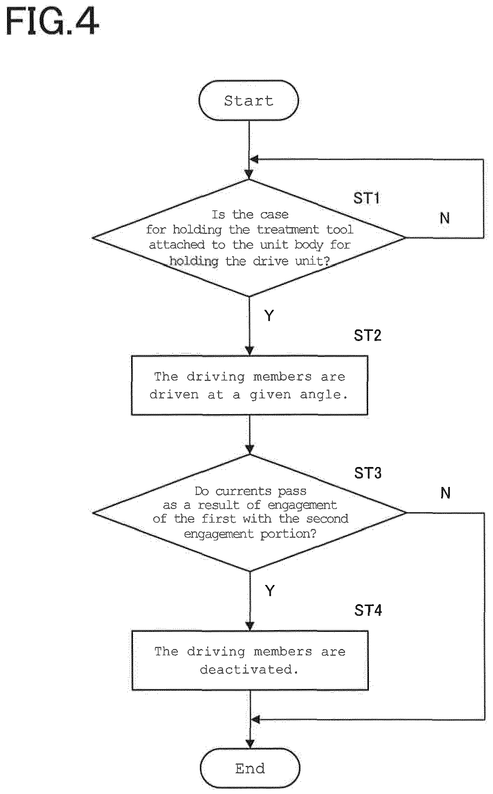

Fig. 4 is a control flowchart for the manipulator system according to one embodiment.

In Step 1, whether or not the case 6 that holds the treatment tool 3 in place is mounted on the unit body 5 that holds the drive unit 2 in place is sensed by the mount sensor unit 8 (ST1). At the time when the case 6 is found to be not mounted in Step 1, the control process goes back to Step 1. At the time when the case 6 is found to be mounted in Step 1, the control process goes to Step 2 in which the driving members 21 of the drive unit 2 are driven (ST2). The driving members 21 are driven until they are rotated by a given angle. The first and second engagement portions 41a and 42a shown in Fig. 2 are formed by 2 on a straight line passing through the center of the surface of the first and second driving force transmission members 41 and 42; for instance, the first and second engagement portions will be engaged with each other if the driving members 21 are rotated by ±90° at most.

Then, the control process goes to Step 3 in which whether or not the first engagement portion 41a is engaged with the second engagement portion 42a is sensed (ST3). In other words, whether or not currents pass through the first and second electrically conductive portions 44 and 45 as a result of their connection are sensed by the current sensor 46.

At the time when currents are not sensed by the current sensor 46 even upon rotated at a given angle in Step 3, the control process gets done, judging that there is a detaching of the treatment tool 3 or other malfunction. At the time when currents are sensed by the current sensor 46 in Step 3, the driving member 21 comes to a stop, finally followed by halting of all the driving members 21.

Some specific examples of the attachment/detachment mechanism 4 in the manipulator system 1 according to the embodiment described here are now explained; for a better understanding, only a set of first and second driving force transmission members 41 and 42 are here explained.

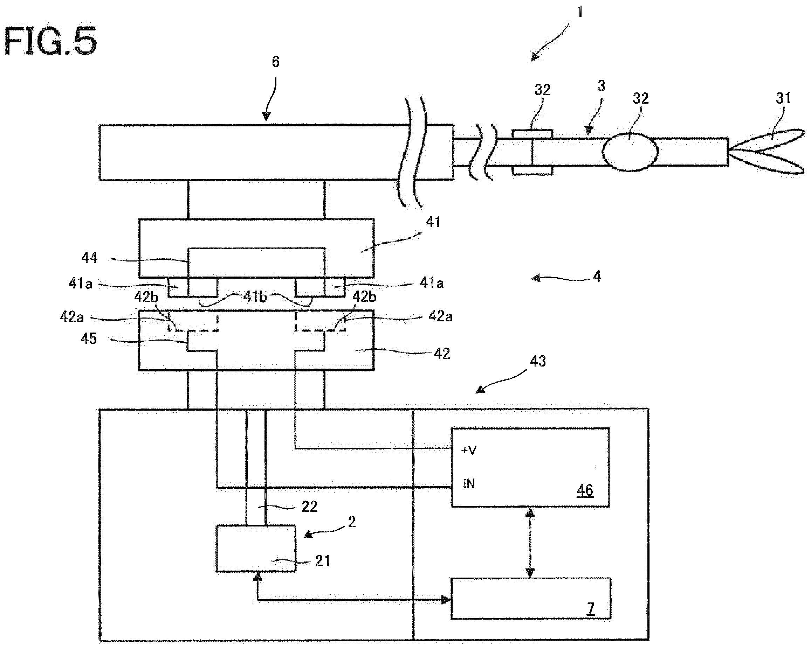

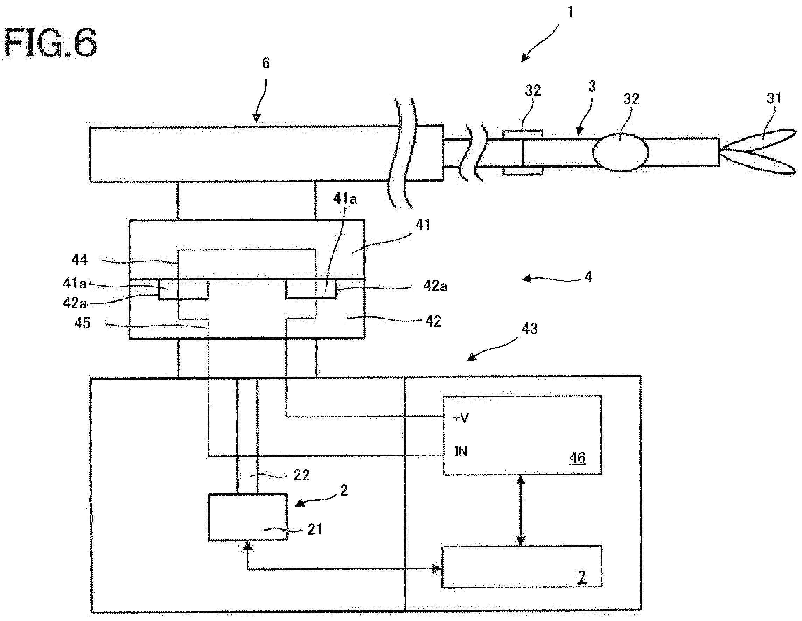

Fig. 5 is illustrative of the manipulator system of the first example according to one embodiment in a disengaged state, and Fig. 6 is illustrative of the manipulator system of the first example according to one embodiment in an engaged state.

In the manipulator system 1 of the first example, there are two first convex engagement portions 41a formed on a straight line passing through the center of the surface of the first driving force transmission member 41 and with that center in between, and there are two second concave engagement portions 42a formed on a straight line passing through the center of the surface of the second driving force transmission member 42 and with that center in between.

Further, the first electrically conductive portion 44 is formed on the top surfaces 41b of the two convex, first engagement portions 41a in the first driving force transmission member 41 in such a way that both its ends are exposed to view, and the second electrically conductive portion 45 is formed on the bottom surfaces 42b of the two concave, second engagement portions 42a in the second driving force transmission member 42 in such a way that one end is exposed to view while another end is connected to the current sensor 46. The current sensor 46 is configured to sense that there is a closed circuit formed as a result of connection of the first and second electrically conductive portions 44 and 45.

The operation of the manipulator system 1 according to the first example is now explained.

In the manipulator system of the first example shown in Fig. 5, the two convex, first engagement portions 41a in the first driving force transmission member 41 and the two concave, second engagement portions 42a in the second driving force transmission member 42 are not always located at an engaging position from the start. In the manipulator system 1, therefore, the driving member 21 of the drive unit 2 is driven to rotate the second driving force transmission member 42 while the first driving force transmission member 41 is pressed against the second driving force transmission member 42, whereupon the first engagement portions 41a are engaged with the second engagement portions 42a halfway in the process of rotation of the second driving force transmission member 42.

Thus, as the two convex, first engagement portions 41a in the first driving force transmission member 41 come into engagement with the two concave, second engagement portions 42a in the second driving force transmission member 42, it causes the first electrically conductive portion 44 to be connected to the second electrically conductive portion 45, resulting in formation of a closed circuit by the first and second electrically conductive portions 44 and 45 and the current sensor 46. If the currents are sensed by the current sensor 46, it then allows the control unit 7 to deactivate the driving member 21. The manipulator system 1 is thus placed in an initial state in which, as shown in Fig. 6, the drive unit 2 remains engaged with the treatment tool 3 and the driving member 21 remains deactivated.

According to the manipulator system 1 of the first example, it is thus possible to achieve unerring engagement of the treatment tool with the drive unit even when they are engaged with each other after insertion of the treatment tool in the body cavity, because the joints of the treatment tool remains fixed after engagement, having no or little influences on the surroundings.

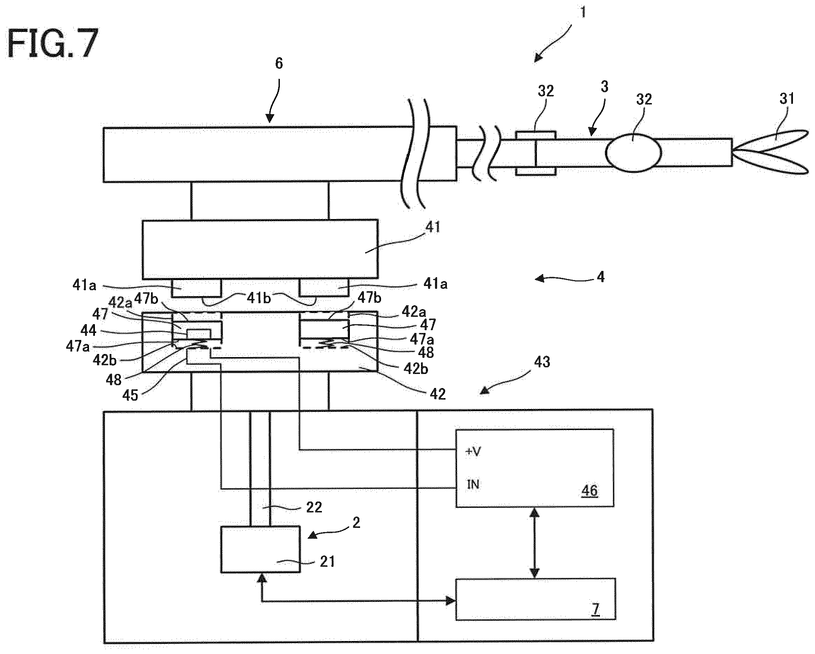

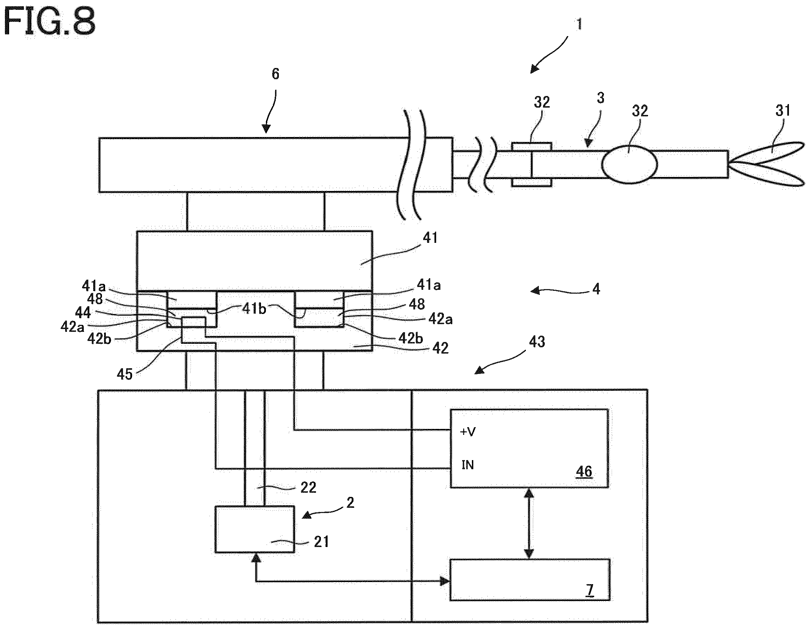

Fig. 7 is illustrative of the manipulator system of the second example according to one embodiment in a disengaged state, and Fig. 8 is illustrative of the manipulator system of the second example according to one embodiment in an engaged state.

In the manipulator system 1 of the second example, there are two first convex engagement portions 41a formed on a straight line passing through the center of the surface of the first driving force transmission member 41 and with that center in between, and there are two second concave engagement portions 42a formed on a straight line passing through the center of the surface of the second driving force transmission member 42 and with that center in between. The manipulator system further includes a moving member 47 located within the second concave engagement portions 42a and an elastic member 48 that is attached at one end to the moving member 47 and at the other end to the bottom surfaces 42b of the second engagement portions 42a. For the second example, it is only required to have a set of the first and second engagement portions 41a and 42a.

Further, the first electrically conductive portion 44 is formed on the first surface 47a of the moving member 47 in the elastic member 48 side in such a way that both its ends are exposed to view, and the second electrically conductive portion 45 is formed on the bottom surfaces 42b of the concave, second engagement portions 42a in the second driving force transmission member 42 in such a way that one end is exposed to view while another end is connected to the current sensor 46. The current sensor 46 is configured to sense that there is a closed circuit formed as a result of connection of the first and second electrically conductive portions 44 and 45.

The operation of the manipulator system 1 according to the second example is now explained.

In the manipulator system of the second example shown in Fig. 7, the two first convex engagement portions 41a in the first driving force transmission member 41 and the two second concave engagement portions 42a in the second driving force transmission member 42 are not always located at an engaging position from the start. In the manipulator system 1, the driving member 21 of the drive unit 2 is driven, while the first driving force transmission member 41 is pressed against the second driving force transmission member 42, to rotate the second driving force transmission member 42, whereupon the first engagement portions 41a are engaged with the second engagement portions 42a halfway in the process of rotation of the second driving force transmission member 42.

The engagement of the first engagement portions 41a with the second engagement portions 42a causes the top surfaces 41b of the first convex engagement portions 41a to urge on the second surface 47b of the moving member 47 against the biasing force of the elastic member 48. Then, the first and second electrically conductive portions 44 and 45 are so connected that the first and second electrically conductive portions 44 and 45 and the current sensor unit 46 define together a closed circuit. As currents are sensed by the current sensor unit 46, it allows the control unit 7 to deactivate the driving member 21. The manipulator system 1 is thus placed in an initial state in which, as shown in Fig. 8, the drive unit 2 remains engaged with the treatment tool 3 and the driving member 21 remains deactivated.

According to the manipulator system 1 of the second example, there is thus no need for providing any electrically conductive portion to the first driving force transmission member 41 on the treatment tool 3 side and, hence, there is the disposal treatment tool 3 growing high in terms of general versatility and going down in terms of cost.

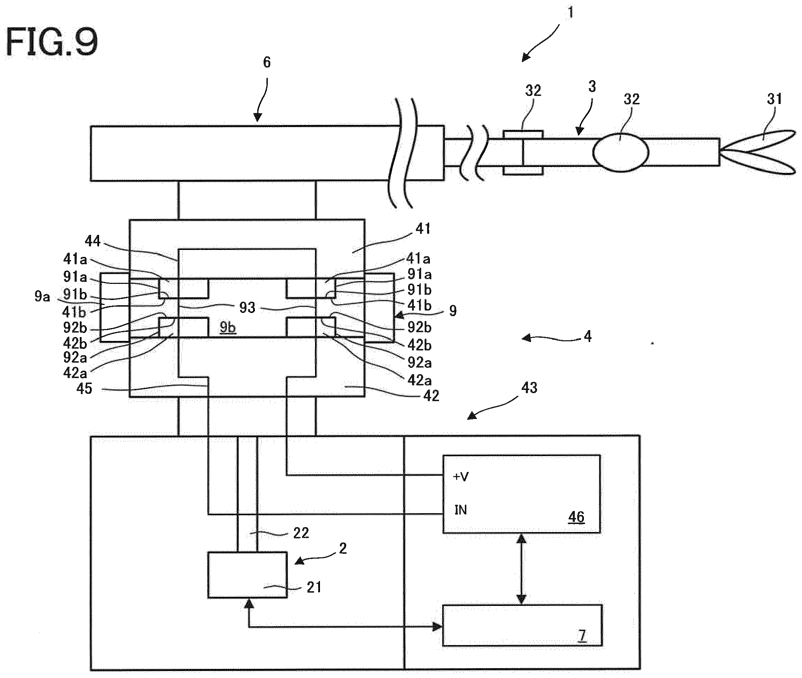

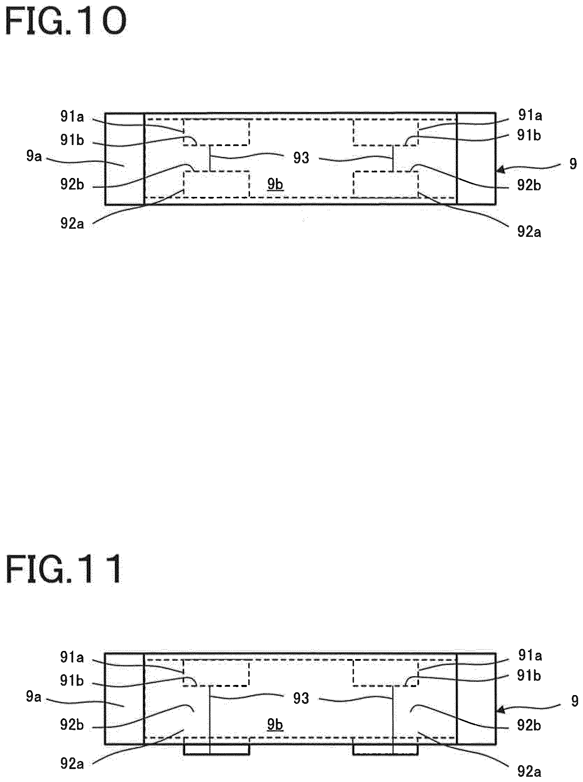

Fig. 9 is illustrative of the manipulator system of the third example according to one embodiment in an engaged state, and Fig. 10 is illustrative of one example of the intermediate member in the manipulator system of the third example according to one embodiment.

In the manipulator system 1 of the third example, there are two first convex engagement portions 41a formed on a straight line passing through the center of the surface of the first driving force transmission member 41 and with that center in between, and there are two second convex engagement portions 42a formed on a straight line passing through the center of the surface of the second driving force transmission member 42 and with that center in between. Further, the manipulator system 1 of the third example includes such an intermediate member 9 as shown in Fig. 10 including a first concave, intermediate engagement portion 91a for engagement with the first convex engagement portion 41a and a second concave, intermediate engagement portion 92a for engagement with the second convex engagement portion 42a and interposed between the first and second driving force transmission members 41 and 42. The intermediate member 9 has an inner member portion 9b supported on an outer member portion 9a in a rotatable manner.

There is a first electrically conductive portion 44 formed on the top surfaces 41b of the two first convex engagement portions 41a in the first driving force transmission member 41 in such a way that both its ends are exposed to view, and there is a second electrically conductive portion 45 formed on the bottom surfaces 42b of the two second concave engagement portions 42a in the second driving force transmission member 42 in such a way that one end is exposed to view and the other ends are connected to the current sensor 46. Two other ends are attached to the positive and negative terminals of the current sensor 46 on which a voltage is applied, respectively. Further, two intermediate, electrically conductive portions 93 are formed on the bottom surfaces 91b and 92b of the two opposite, first and second intermediate engagement portions 91a and 92a in such a way that both ends of each conductive portion are exposed to view.

The operation of the manipulator system 1 of the third embodiment is now explained.

As shown in Fig. 9, it is preferable for the manipulator system 1 of the third example that the intermediate member 9 has previously been engaged with any one of the first and second driving force transmission members 41 and 42.

Referring typically to the case where the intermediate member 9 has previously been engaged with the first driving force transmission member 41, the two second concave, intermediate portions 92a in the intermediate member 9 and the two second convex engagement portions 42a in the second driving force transmission member 42 are not always in an intermediate member 9's engaged position from the start. In this case, the manipulator system 1 first causes the driving member 21 of the drive unit 2 to be driven, while the first driving force transmission member 41 and intermediate member 9 are pressed against the second driving force transmission member 42, to rotate the second driving force transmission member 42, whereupon the second intermediate engagement portion 92a is engaged with the second engagement portion 42a halfway in the process of rotation of the second driving force transmission member 42.

Referring then to the case where the intermediate member 9 has previously been engaged with the second driving force transmission member 42, the two second concave, intermediate portions 92a in the intermediate member 9 and the two first convex engagement portions 41a in the first driving force transmission member 41 are not always in an intermediate member 9's engaged position from the start. In this case, the manipulator system 1 first causes the driving member 21 of the drive unit 2 to be driven, while the first driving force transmission member 41 is pressed against the intermediate member 9, to rotate the second driving force transmission member 42, whereupon the first driving force transmission member 41 is engaged with the first intermediate engagement portion 91a halfway in the process of rotation of the second driving force transmission member 42.

By the engagement of the first engagement portion 41a with the first intermediate engagement portion 91a and the second engagement portion 42a with the second intermediate engagement portion 92a, the first and second electrically conductive portions 44 and 45 and the intermediate electrically conductive portion 93 are so connected that there is a closed circuit formed by the first and second electrically conductive portions 44 and 45, intermediate electrically conductive portion 93 and current sensor 46. As currents are sensed by the current sensor 46, it allows the control unit 7 to deactivate the driving member 21. The manipulator system 1 is thus placed in an initial state in which, as shown in Fig. 9, the drive unit 2 remains engaged with the treatment tool 3 and the driving member 21 remains deactivated.

According to the manipulator system 1 of the third example, it is thus also possible to be compatible with a specific structure in which there is the intermediate member 9 used for the purpose of separating the attachment/detachment mechanism into a clean area and an unclean area.

Fig. 11 is illustrative of another example of the intermediate member in the manipulator system of the third example according to one embodiment.

The intermediate member 9 shown typically in Fig. 11 is used with the first driving force transmission member 41 including the first convex engagement portion 41a and the second driving force transmission member 42 including the second concave engagement portion 42a used in the first and second examples.

More specifically, the intermediate member 9 here includes a first concave, intermediate engagement portion 91a for engagement with the first convex engagement portion 41a and a second convex, intermediate engagement portion 92a for engagement within the second concave engagement portion 42a, and is located between the first and second driving force transmission members 41 and 42. Note here that there is no explanation of other structures provided because they are much the same as explained with reference to Fig. 10.

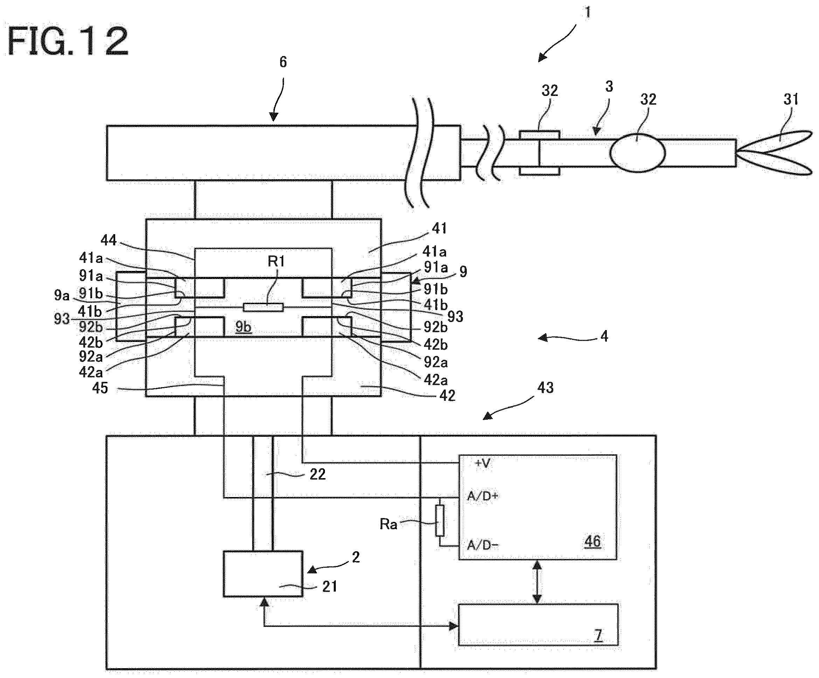

Fig. 12 shows the manipulator system of the fourth example according to one embodiment in an engaged state.

In the manipulator system 1 of the fourth example, the two intermediate electrically conductive portions 93 located in the intermediate member 9 of the third example, as shown in Fig. 9, are connected together via a first resistance R1, and a voltage is applied on one of two other ends of the second electrically conductive portion 45, with the rest being connected to the positive terminal A/D+ of an A/D converter in the current sensor 46 and to the negative terminal A/D- via a sensing resistance Ra. Note here that there is no explanation of other structures provided because they are much the same as those in the third example explained with reference to Fig. 9.

In such arrangement, the voltage measured by the current sensor 46 varies depending on what state it is connected to the drive unit 2 in, as can be seen from the following Table 1. Referring typically to the case where there is no intermediate member 9 connected to the drive unit 2, the voltage will be zero, and referring to the case wherein only the intermediate member 9 is connected to the drive unit 2, the voltage will be (V×Ra)/(R1+Ra). In the case where the intermediate member 9 and treatment tool 3 are connected to the drive unit 2, the voltage will be V.

**Table 1**

| Attachment | Voltage (V) |
|---|---|
| None | 0 |
| Intermediate Member | (V×Ra)/(R1+Ra) |
| Intermediate Member + Treatment Tool | V |

Thus, the voltage of the current sensor 46 may be measured thereby recognizing the state of the treatment tool 3 and intermediate member 9 connected to the drive unit 2.

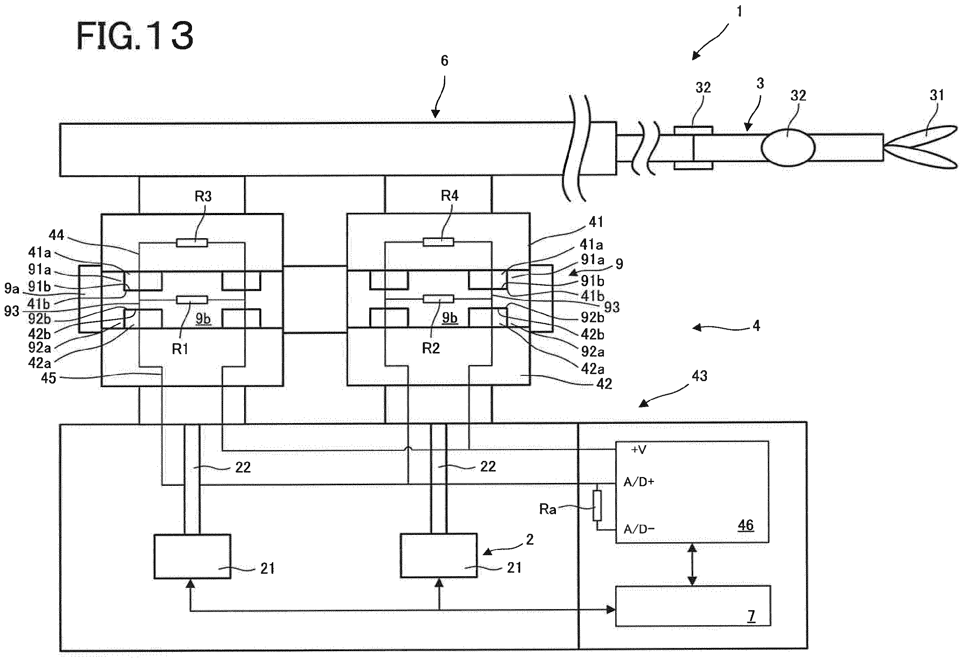

Fig. 13 is illustrative of the manipulator system of the fifth example according to one embodiment in an engaged state.

According to the manipulator system 1 of the fifth example, the technology of recognizing the state of the treatment tool 3 and intermediate member 9 being connected to the drive unit 2 as in the fourth example is applied to the attachment/detachment mechanism 4 including two sets of the first and second driving force transmission members 41 and 42 and intermediate members 9. In the manipulator system 1 of the fifth example, the two intermediate electrically conductive portions 93 located on the two intermediate members 9 are connected together via first and second resistances R1 and R2, and third and fourth resistances R3 and R4 are inserted through the first electrically conductive portions 44 located in the two first driving force transmission members 41 to apply voltage to one of two other ends of the second electrically conductive portion 45 and connect the rest to the positive terminal A/D+ of the A/D converter of the current sensor 46 and the negative terminal A/D- via a sensing resistance Ra. Note here that there is no explanation of other structures provided because of being much the same as in third example shown in Fig. 9.

In such arrangement, the voltage measured by the current sensor 46 varies depending on what state it is connected to the drive unit 2 in, as can be seen from the following Table 2. Referring typically to the case where only the first set of intermediate members is connected to the first set of driving members 21, the voltage will be (V×Ra)/(R1+Ra), and in the case where all of the first and second sets of intermediate members 9 and treatment tool 3 are connected to the drive unit 2, the voltage will be (V×Ra)/((R6×R7)/(R6+R7)+Ra) with the proviso that R6=(R1×R3)/(R1+R3 ) and R7=(R2×R4)/(R2+R4).

**Table 2**

| Attachment | Voltage (V) |
|---|---|
| (1) | (V×Ra)/(R1+Ra) |
| (2) | (V×Ra)/(R2+Ra) |
| (3) | (V×Ra)/((R1×R2)/(R1+R2)+Ra) |
| (4) | (V×Ra)/((R6×R2)/(R6+R2)+Ra) provided that R6=(R1×R3)/(R1+R3) |
| (5) | (V×Ra)/((R1×R7)/(R1+R7)+Ra) provided that R7=(R2×R4)/(R2+R4) |
| (6) | (V×Ra)/((R6×R7)/(R6+R7)+Ra) |

| | |
|---|---|
| (1) Only the driving force transmission member 1 for the intermediate member (2) Only the driving force transmission member 2 for the intermediate member (3) All the driving force transmission members for the intermediate member (4) All the driving force transmission members for the intermediate member plus the driving force transmission member 1 for the treatment tool (5) All the driving force transmission members for the intermediate members plus the driving force transmission member 2 for the treatment tool (6) All the driving force transmission members for the intermediate member plus all the driving force transmission members for the treatment tool | |

Thus, the voltage of the current sensor 46 may be measured for separate sensing of the state of the treatment tool 3 being engaged with the intermediate member, and the state of the intermediate member 9 being engaged with the drive unit 2. Use of the A/D convertor for each engagement portion also makes it possible to reduce the number of A/D convertors used.

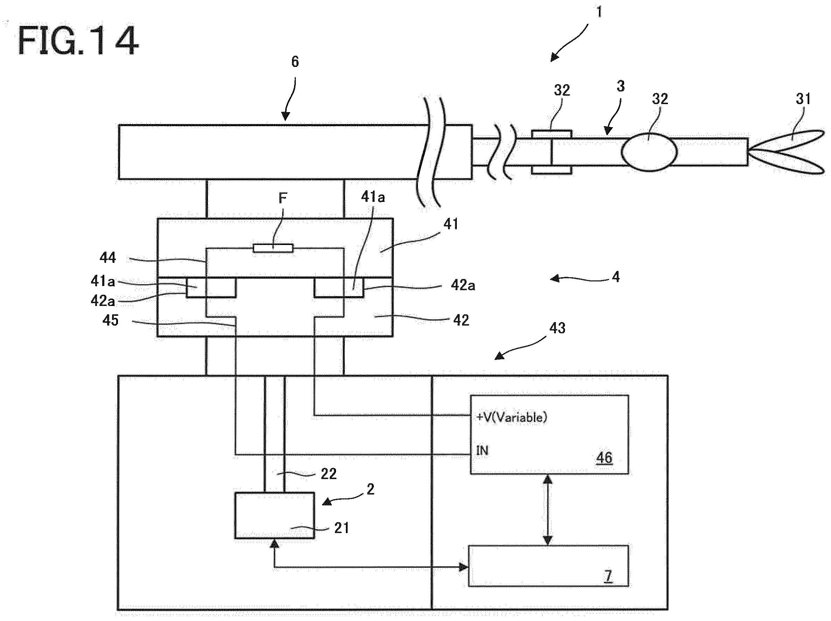

Fig. 14 is illustrative of the manipulator system of the sixth example according to one embodiment in an engaged state.

In the manipulator system 1 of the sixth example, there is a fuse resistor F inserted through the first electrically conductive portion 44 mounted on the first driving force transmission member 41 provided for the treatment tool 3 of the first example shown in Fig. 6, and as the treatment tool 3 reaches a predetermined frequency in use, it causes the voltage applied on the fuse resistor F to rise high enough to open or burn the fuse resistor F. How often the treatment tool 3 is used may be counted by using a reader for reading out code data or the like affixed to the treatment tool 3. Note here that there is no explanation of other structures provided because of being much the same as in the first example shown in Fig. 6.

Thus, the fuse resistor F may be used to place limitation on the frequency of the treatment tool 3 in use.

The medical system 100 to which the manipulator according to one embodiment is applied will now explained.

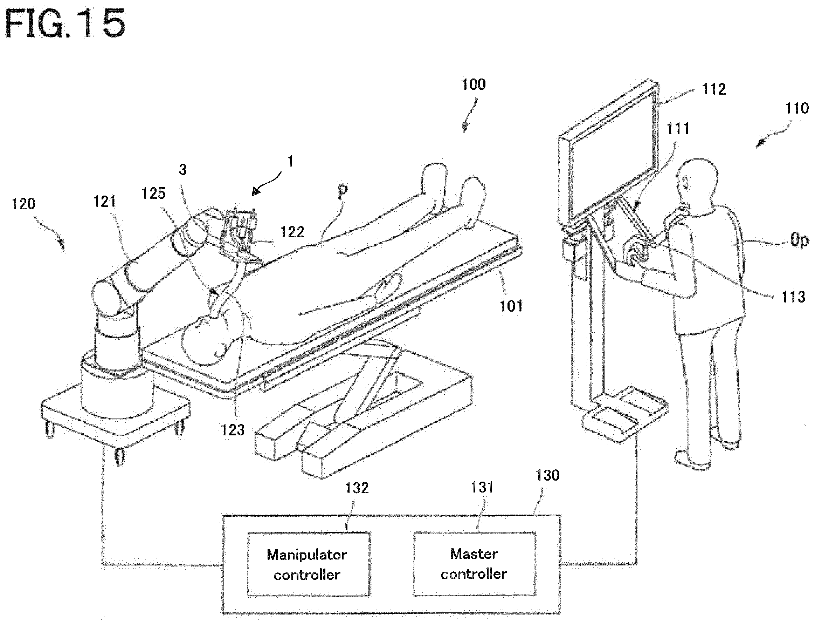

Fig. 15 is illustrative of the medical system 100 to which the manipulator according to one embodiment is applied.

The medical system 100 according to one embodiment preferably operates on a master slave mode. The medical system 100 includes a master input 110 that includes a master arm 111 for producing out an operation command, and a slave manipulator 120 including a slave arm 121 wherein the slave arm 121 is remotely controlled in such a way as to keep track of the operation of the master arm 111 by a practitioner (or operator). The operation command entered via the master arm 111 is transmitted to the master controller 131 in the system control unit 130, and entered into the manipulator controller 132, optionally after subjected to the necessary transformation processing. After that, an operating signal is sent from the manipulator controller 132 to the slave manipulator 120 for activation of the slave arm 121.

As shown in Fig. 15, the slave manipulator 120 is mounted on an operating table 101 on which a patient P lies down. The slave arm 121 is in an assembly form having a plurality of joints having multiple degrees of freedom, and capable of multi-axis movements. The respective joints having multiple degrees of freedom are each independently driven by a power source, not shown. For instance, the power source used may be a motor (servo motor) having a servo mechanism including an incremental encoder or a decelerator.

The slave arm 121 is mounted at its distal end with a manipulator 1 that is inserted through the body cavity of the patient P for surgical techniques and an endoscope 122. The treatment tool 3 and endoscope 122 are inserted through an overtube 123, defining an insert assembly 125. The distal end of the overtube 123 is inserted through the body cavity of the patient P. The treatment tool 3 in an assembly form includes a plurality of distal tool portions that vary in structure and shape so as to be compatible with various surgical techniques, and they are replaced and mounted on the distal end of the slave arm 121 for various surgical techniques. The endoscope 122 gains images of an operating field inclusive of a site to which a given surgical technique is applied by the treatment tool 3 in the body cavity of the patient P.

The master input 110 includes a plurality of master arms 111 operated by the practitioner Op and a display 112 for showing images gained through the endoscope 122. Each or the master arm 111 has a known arrangement capable of multi-axis movements, and includes a grip 113 positioned the distal end side of near the practitioner Op and working as an operating portion held by the practitioner to produce out an operation command.

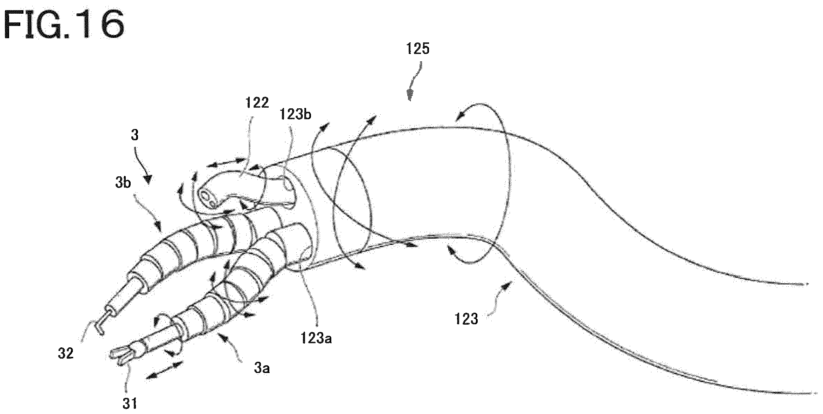

Fig 16 is schematically illustrative of the structure of the insert assembly 125 according to one embodiment.

The insert assembly 125 here includes a flexible, elongate overtube 123, a treatment tool 3 inserted in an insert opening 123a in the overtube 123, and an endoscope 122 inserted in an insert opening 123b in the overtube 123. Note here that the treatment tool 3 has a structure applicable to the manipulator system 1 according to one embodiment.

The treatment tool 3 of the embodiment here, in an assembly form, includes a first treatment tool 3a and a second treatment tool 3b and, as end effectors, the first treatment tool 3a includes a grip 31 and the second treatment tool 3b includes an electrically-operated knife 32. The distal end side of the treatment tool 3 is projectable out of the overtube 123, and constructed of a bendable assembly in which there are a plurality of joint rings lined up in an axial direction. The most distal-end joint ring is fixedly provided with both ends of an operating wire for driving the bendable assembly so that the operating wire can be driven to bend the bendable assembly and rotate it in the axial direction as well.

The distal end of the endoscope 122 is also projectable out of the overtube 123, and a flexible, tubular member is driven by the operating wire to bend and rotate it in the axial direction. Likewise, the overtube 123 per se, too, is preferably bendable and rotatable in the axial direction.

As described above, the manipulator system 1 here includes a drive unit 2 including a plurality of driving members 21 for generating driving force, a treatment tool 3 that is driven by the drive unit 2, an attachment/detachment mechanism 4 for attaching and detaching the drive unit 2 on and out of the treatment tool 3, and a control unit 7 for gaining control of the drive unit 2, wherein the attachment/detachment mechanism 4 includes a plurality of first driving force transmission members 41 which are attached on the treatment tool 3 and to which associated driving forces are transmitted from the plurality of driving members 21, a plurality of second driving force transmission members 42 which are attached on the driving members 21 and transmit associated driving forces to the plurality of first driving force transmission members 41, and an engagement sensor unit 43 that is capable of separately sensing attaching and detaching of a first engagement portion 41a formed on each of the plurality of first driving force transmission members 41 on and out of a second engagement portion 42a formed on each of the plurality of second driving force transmission members 42, and the control unit 7 is configured such that when the engagement sensor unit 43 senses engagement of the first engagement portion 41a with the second engagement portion formed 42a, there is deactivation of the driving member 21 for driving the second driving force transmission member 42 of the plurality of second driving force transmission members 42, which member has the sensed second engagement portion 42a. It is thus possible to achieve unerring engagement of the treatment tool with the drive unit even when the treatment tool is engaged with the drive unit after insertion of the treatment tool in the body cavity, because the joints of the treatment tool remain immobilized with no or influences on the surroundings.

The manipulator system 1 herein described includes a unit body 5 for holding the drive unit 2, a case 6 for holding the treatment tool 3, and a mount sensor unit 8 for sensing mounting of the case 6 on the unit body 5, wherein the control unit 7 is configured such that when the mount sensor unit 8 senses mounting of the case 6 on the unit body 5, the drive unit 2 is activated. It is thus possible to rotate the second driving force transmission members 42 while the first engagement portions 41a are pressed against the second driving force transmission members 42.

In the manipulator system 1 here, the attachment/detachment mechanism 4 further includes an intermediate member 9 that includes a first intermediate engagement portion 91a for engagement with the first engagement portion 41a and a second intermediate engagement portion 92a for engagement with the second engagement portion 42a and is attachably/detachably located between the first driving force transmission members 41 and the second driving force transmission members 42. Even with the intermediate member 9, it is thus possible to sense the engagement.

In the manipulator system 1 here, the engagement sensor unit 43 includes a first electrically conductive portion 44 located in the first engagement portion 41a, a second electrically conductive portion 45 located in the second engagement portion 42a, and a current sensor unit 46 for sensing currents through the first and second electrically conductive portions 44 and 45. It is thus possible to unerringly sense the engagement.

In the manipulator system 1 here, it is possible to place limitation of the frequency of the treatment tool 3 in use, because there is a fuse inserted through the first electrically conductive portion 44.

In the manipulator system 1 here, the intermediate member 9 further includes a third electrically conductive portion 93 interposed between the first and second intermediate engagement portions 91a and 92a, and when the first engagement portion 41a engages with the first intermediate engagement portion 91a and the second engagement portion 42b engages with the second intermediate engagement portion 92a, it causes the third electrically conductive portion 93 to make a connection between the first and second electrically conductive portions 44 and 45. It is thus possible to improve on the degree of design freedom and achieve unerring sensing of the engagement.

In the manipulator system 1 here, the intermediate member 9 further includes a resistance inserted between two third electrically conductive portions 93 interposed between at least two first intermediate engagement portions 91a and at least two second intermediate engagement portions 92a, and a voltage is applied on one of the second electrically conductive portions 45 on the current sensor unit 46 and the other is connected to the positive and negative terminals of an A/D convertor with a sensing resistance Ra inserted between the positive terminal and the negative terminal. It is thus possible to sense the intermediate member 9 and treatment tool 3 in a distinct way.

In the manipulator system 1 here, the engagement sensor unit 43 includes a moving member 47 located within the second engagement portion 42a, an elastic member 48 for supporting the moving member 47 relative to the second driving force transmission members 42, a first electrically conductive portion 44 located in the moving member 47, a second electrically conductive portion 45 located in the second engagement portion 42a, and a current sensor unit 46 for sensing a current passing through the first and second electrically conductive portions 44 and 45. It is thus possible to make the general versatility of the treatment tool 3 and the cost of the disposal treatment tool 3 low because there is no need for providing any electrically conductive portion to the first driving force transmission members 41 on the treatment tool 3 side.

The medical system 100 according to one embodiment includes a master input 110 including a master arm 111 for issuing an operation command, and a slave manipulator 120 including a slave arm 121 and a manipulator system 120, wherein the slave arm 121 is remotely controlled in such a way as to keep track of operation of the master arm 111. It is thus possible to hold back influences on the surroundings and achieve unerring engagement of the treatment tool with the drive unit even when the treatment tool is engaged with the drive unit after insertion of the treatment tool through the body cavity.

It is here to be appreciated that the invention is in no sense limited to such embodiments as described above. While the explanation of some embodiments embraces numerous specific details for illustration, it would be obvious to those skilled in the art that diverse variations or modifications made thereto are included within the scope of the invention. In other words, illustrative embodiments of the invention are described without excluding generality from the claimed inventions and imposing any limitation thereon.

### Reference Signs List

1: Manipulator system
2: Drive unit
21: Driving member
22: Output shaft
3: Treatment tool
4: Attachment/detachment mechanism
41: First driving force transmission member
41a: First engagement portion
42: Second driving force transmission member
42a: Second engagement portion
43: Engagement sensor unit
44: First electrically conductive portion
45: Second electrically conductive portion
46: Current sensor
47: Moving member
48: Elastic member
5: Unit body
6: Case
7: Control unit
8: Mount sensor unit
9: Intermediate member
91a: First intermediate engagement portion
92a: Second intermediate engagement portion
93: Intermediate, electrically conductive portion
100: Medical system

## Claims

1. A manipulator system, **characterized by** comprising:
a drive unit including a plurality of driving members for generating driving force,
a treatment tool that is driven by the drive unit,
an attachment/detachment mechanism for attaching and detaching the drive unit on and out of the treatment tool, and
a control unit for gaining control of the drive unit, **characterized in that**:
the attachment/detachment mechanism comprises:
a plurality of first driving force transmission members which are attached on the treatment tool and to which associated driving forces are transmitted from the plurality of driving members,
a plurality of second driving force transmission members which are attached on the driving members and which transmit associated driving forces to the plurality of first driving force transmission members, and
an engagement sensor unit that is capable of separately sensing attaching and detaching of a first engagement portion formed on each of the plurality of first driving force transmission members on and out of a second engagement portion formed on each of the plurality of second driving force transmission members, and
the control unit is configured such that when the engagement sensor unit senses engagement of the first engagement portion formed on each of the plurality of first driving force transmission members with the second engagement portion formed on each of the plurality of second driving force transmission members, there is deactivation of the driving member for driving the second driving force transmission member of the plurality of second driving force transmission members, which member has the sensed second engagement portion.

2. A manipulator system according to claim 1, which comprises:
a unit body for holding the drive unit,
a case for holding the treatment tool, and
a mount sensor unit for sensing mounting of the case on the unit body, wherein:
the control unit is configured such that when the mount sensor unit senses mounting of the case on the unit body, the drive unit is activated.

3. A manipulator system according to claim 1 or 2, wherein the attachment/detachment mechanism comprises an intermediate member that includes a first intermediate engagement portion for engagement with the first engagement portion and a second intermediate engagement portion for engagement with the second engagement portion and is attachably/detachably located between the first driving force transmission members and the second driving force transmission members.

4. A manipulator system according to any one of claims 1, 2 and 3, the engagement sensor unit comprises:
a first electrically conductive portion located in the first engagement portion,
a second electrically conductive portion located in the second engagement portion, and
a current sensor for sensing currents through the first and second electrically conductive portions.

5. A manipulator system according to any one of claims 1 to 4, wherein there is a fuse inserted through the first electrically conductive portion

6. A manipulator system according to claim 5, wherein the intermediate member further includes a third electrically conductive portion interposed between the first and second intermediate engagement portions, and when the first engagement portion engages with the first intermediate engagement portion and the second engagement portion engages with the second intermediate engagement portion, it causes the third electrically conductive portion to make a connection between the first and second electrically conductive portions.

7. A manipulator system according to claim 6, wherein the intermediate member further includes a resistance inserted between two third electrically conductive portions interposed between at least two first intermediate engagement portions and at least two second intermediate engagement portions, and
a voltage is applied on one of the second electrically conductive portions on the current sensor unit side and the other is connected to a positive terminal and a negative terminal of an A/D convertor with a sensing resistance Ra inserted between the positive terminal and the negative terminal.

8. A manipulator system according to claim 1 or 2, wherein the engagement sensor unit comprises:
a moving member located within the second engagement portion,
an elastic member for supporting the moving member relative to the second driving force transmission members,
a first electrically conductive portion located in the moving member,
a second electrically conductive portion located in the second engagement portion, and
a current sensor for sensing a current passing through the first and second electrically conductive portions.

9. A medical system, comprising:
a master input including a master arm for issuing an operation command, and
a slave manipulator including a slave arm and a manipulator system according to any one of claims 1 to 8, wherein the slave arm is remotely controlled in such a way as to keep track of operation of the master arm.
